# FASCICULE DE BREVET EUROPEEN

(11) **EP 1 100 482 B1**
(45) Date de publication et mention de la délivrance du brevet: **23.07.2003**
(21) Numéro de dépôt: 99936679.2
(22) Date de dépôt: 03.08.1999
(51) Int. Cl.: A61K 31/05, A61K 31/18, A61K 31/66, A61K 31/74, A61P 17/02

(54) **UTILISATION DES CALIX(N)ARENES POUR LA FABRICATION D'UN MEDICAMENT POUR LE TRAITEMENT DES MALADIES FIBROTIQUES**
VERWENDUNG VON CALIX(N)ARENEN ZUR HERSTELLUNG EINES MEDIKAMENTES ZUR BEHANDLUNG VON FIBROTISCHEN KRANKHEITEN
USE OF CALIX(N)ARENES IN THE MANUFACTURE OF A MEDICAMENT FOR TREATING FIBROTIC DISEASES

(30) Priorité: 05.08.1998 FR 9810074
(43) Date de publication de la demande: 23.05.2001
(73) Titulaire: CENTRE NATIONAL DE LA RECHERCHE SCIENTIFIQUE, 75016 Paris Cédex (FR)
(72) Inventeur: HULMES, David, F-69230 Saint Genis-Laval (FR); COLEMAN, Antony, 69000 Caluire (FR); AUBERT-FOUCHER, Elisabeth, F-69002 Lyon (FR)
(74) Mandataire: Catherine, Alain
(86) Numéro de dépôt international: FR9901922
(87) Numéro de publication internationale: WO00007585

(56) Documents cités:
- EP-A- 0 374 440
- WO-A-94/03165
- WO-A-96/40746
- US-A- 4 997 854

## Description

La présente invention a pour objet l'utilisation des calix(n)arènes pour la Fabrication d'un médicament pour le traitement des maladies fibrotiques.

Les tissus conjonctifs (os, cartilage, peau, tendon etc...) sont essentiels à l'intégrité structurale des vertébrés, les molécules de la matrice extracellulaire assurant le soutien des tissus et organes.

La matrice extracellulaire a de nombreuses implications en pathologie incluant plusieurs maladies fibrosantes du foie, du rein, du poumon et de la peau où son dépôt excessif et ectopique entraîne les phénomènes de cicatrisation, et/ou nécrose et des dysfonctionnements organiques. Les composants macromoléculaires de la matrice extracellulaire comprennent les collagènes, les protéoglycanes, l'élastine. d'autres glycoprotéines structurales, des enzymes extracellulaires et des facteurs de croissance, la plupart d'entre eux interagissant avec une grande diversité de récepteurs de la surface cellulaire. L'assemblage des collagènes et de l'élastine en fibres insolubles, responsables de l'intégrité structurale des tissus conjonctifs est stabilisé par la formation de liens covalents intra- et inter-moléculaires. La formation de ces liens covalents est assurée par l'action d'une enzyme extracellulaire, la lysyl-oxydase et par une glycosylation non enzymatique.

La réticulation initiée par la lysyl oxydase est un facteur essentiel dans les pathologies fibrosantes du foie (**Konishi et al.**, (1985) Gastroenterol. 89, 709-715; **Wakasaki et Ooshima,** 1990, Biochem. Biophys. **Res.** Commun., 166,1201-1204, **Murawaki et al.,** 1991, Hepatology 14, 1167-1173; Sommer et al.,1993 Lab. Invest. 69, 460-470, **Ricard-Blum et al.,** 1993 Cell. Mol. Biol. 39, 723-727 ) de la peau (**Ricard-Blum et al** 1996 Gastroenterol.111, 172-182 ), du poumon (**Counts et al**., 1981 Pharmacol. Exp. Therap.219, 675-678 ; **Hameleinen et al.,** 1995, 270, 21590-21593 ) et toutes les autres formes de fibrose (**Ma et al.,** (1995) J. Oral Pathol. Med.24, 407-412). Elle joue également un rôle important dans la réaction stromale et intervient de ce fait dans l'invasion tumorale (**Peyrol et al.,** 1997 Amer. J. Pathol. 150, 497-507 ).

La lysyl oxydase est sécrétée dans la matrice extracellulaire sous forme d'un précurseur de 50 Kda et transformée en protéine mature de 30 kDa par coupure enzymatique de la partie N-terminale du pro-peptide . Quatre variants de l'enzyme ont été décrits. Cette enzyme a été isolée et caractérisée à partir de divers tissus conjonctifs (**Kagan**, Biology of extracellular matrix, 321-398 1986; **Kagan et Trackman**, Am. J. respir. Cell. Mol. Biol, 5, 206-210, 1991).

Des inhibiteurs spécifiques de la lysyl oxydase ont été décrits tels que le β amino-propionitrile (βAPN; Kᵢ= 6 µM) (**Kagan,** 1994 précédemment cité). Malheureusement, l'utilisation du βAPN dans le traitement des maladies fibrosantes est exclue en raison de sa toxicité . Diverses diamines ont aussi été décrites comme inhibiteurs de la lysyl oxydase (**Gacheru et al.** J. Biol. Chem.264, 12963-12969 (1989)).

Les calix(n)arènes sont une catégorie d'oligophénols cycliques provenant de la réaction entre des phénols para-substitués et des formaldéhydes en présence d'une base. Ils ont récemment été décrits comme pouvant être utilisés pour le traitement des thromboses, et comme agents antiviraux.

Ainsi, le brevet US 5 409 959 décrit une méthode de traitement de la thrombose consistant à administrer par la voie intra-veineuse ou subcutanée ou par inhalation une quantité efficace de calix(n)arènes.

Le brevet US 5 441 983 quant à lui décrit l'administration de calix(n)arènes pour traiter des infections dues à des virus à enveloppe tels que les virus de l'herpès ,VSH-1 ou VSH-2. L'administration par injection et l'administration topique de ces composés sont décrites.

Les inventeurs ont montré qu'il était possible de traiter les maladies fibrotiques par des calix(n)arènes.

La présente invention est donc relative à l'utilisation des calix(n)arènes pour la fabrication d'un médicament pour le traitement des maladies fibrotiques.

On entend par maladie fibrotique selon la présente invention, toute pathologie des tissus fibreux générant une fibrose, et en particulier des pathologies aboutissant à une dégénérescence des tissus fibreux.

De telles pathologies peuvent être:
* les fibroses aiguës dues à des traumas:
   - cicatrisation suite à des opérations chirurgicales (de l'abdomen, des yeux, des mains, de la colonne vertébrale, ainsi qu'arthroplastie et angioplastie),
   - infections (fibrose pulmonaire)
   - blessures (mains)
   - brûlures (cicatrisation hypertrophique)
   - traitements par radiations ionisantes et chimiothérapie
   - traitements avec les lasers (cornée)
* les fibroses chroniques des organes majeurs:
   - foie
   - poumons
   - reins
   - coeur
   - peau
* d'autres maladies fibro-prolifératives:
   - sclérodermie
   - pathologies chéloïdes
   - athérosclérose

La présente invention est particulièrement adaptée au traitement topique de la peau, notamment la cicatrisation de la peau.

Préférentiellement, les calix(n)arènes sont choisis pour leur capacité à inhiber substantiellement l'activité lysyl oxydase. De manière avantageuse, ces molécules présentent une concentration inhibant à 50% l'activité lysyl-oxydase (IC50) inférieure à 100 mM et préférentiellement inférieure à 10 mM.

Les calix(n)arènes particulièrement adaptés au traitement des maladies fibrotiques, sont ceux présentant la formule I suivante: 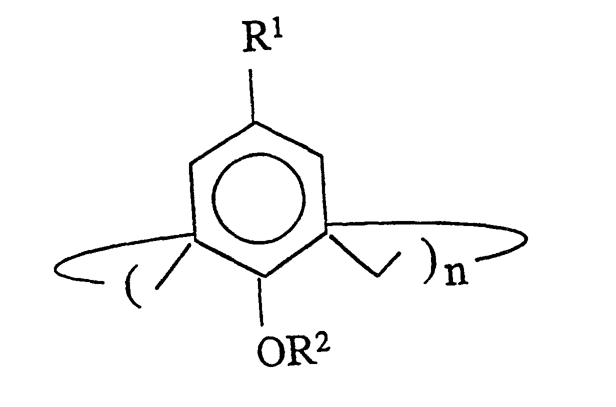 dans laquelle:
- R₁ représente un groupe alkyle, alkylamide, acyle, aryle, carboxyle, sulfonyle ou phosphonyle, éventuellement substitué
- R₂ représente un atome d'hydrogène, un groupe alkyle, un groupe acyle, ou un groupe amide, éventuellement substitué,
- n est un nombre entier compris entre 4 et 8, et leurs sels et esters pharmaceutiquement acceptables.

Les groupes alkyle et acyle sont préférentiellement des groupes en C₁ à C₁₀, encore plus préférentiellement en C₂ à C₆, éventuellement substitués.

Préférentiellement, le groupe aryle est un groupe en C₅ à C₂₀ , encore plus préférentiellement en C₅ à C₁₀. Il est avantageusement un groupe phényle, biphényle, ou phényle substitué par un groupe carbohydrate.

Un tel carbohydrate peut être un résidu glycosyle, maltosyle, mannosyle ou galactosyle.

Avantageusement les groupes phényles sont substitués en position para.

Selon un mode de mise en oeuvre préférentiel de l'invention, le groupe R₁ représente un groupe SO₃H, éventuellement sous forme de sel.

De manière préférentielle, n est égal à 6.

Un composé pouvant être utilisé de manière particulièrement avantageuse pour traiter les maladies fibrotiques est celui dans lequel n est égal à 6, R₁ représente un groupe SO₃H et R₂ un atome d'hydrogène.

Préférentiellement, les calix(n)arènes sont mis sous la forme de sels de lithium, de sodium, de potassium, de rubidium ou de césium.

Les composés répondant à la formule générale I peuvent être fabriqués comme décrits par **ARENA et al.**(1992, Supramolecular Chemistry 1, 19-24) par réaction de phénols substitués en position para par le groupe R₁ d'un aldéhyde en présence d'une base, suivie d'une terbutylation et d'une substitution électrophile en présence de H₂SO₄.

Certains de ces composés sont commercialisés. En particulier, le calix(6)arène dans lequel n est égal à 6, R₁ représente SO₃H et R₂ un atome d'hydrogène , qui constitue un mode de mise en oeuvre préférentiel de l'invention est commercialisé par la Société ACROS (Geel West - Belgique) sous la référence 29 026-0010.

Les voies d'administration de ces composés dépendent des pathologies à traiter. Ces modes d'administrations sont déterminés par l'homme du métier.

Une méthode de traitement des maladies fibrotiques comprend en substance une étape d'administration au sujet à traiter d'une dose thérapeutiquement efficace d'un ou plusieurs calix-n-arènes. Cette administration peut être effectuée de manière topique s'il s'agit par exemple d'une maladie de peau.

Elle peut être néanmoins effectuée de toute manière connue de l'homme du métier, et notamment par injection, de manière sous-cutanée, ou par inhalation.

Les doses de calix(n)arènes administrées aux patients sont à évaluer en fonction de la pathologie à traiter, et de divers autres paramètres connus de l'homme du métier, tel que le poids du patient.

A titre indicatif, le traitement de maladies fibrotiques cutanées est effectué par application d'une dose comprise entre 1 µg et 1g de calix(n)arènes, dans un véhicule approprié.

Un avantage particulier des calix(n)arènes est leur faible toxicité par rapport aux inhibiteurs connus de la lysyl-oxydase.

La présente invention est en outre relative à un procédé in vitro d'inhibition de l'activité lysyl oxydase comprenant la mise en contact de la lysyl oxydase et d'un calix(n)arène. Elle a aussi pour objet un procédé in vitro d'utilisation des calix(n)arènes pour inhiber l'activité lysyl oxydase.

La présente invention est illustrée sans pour autant être limitée par l'exemple qui suit.

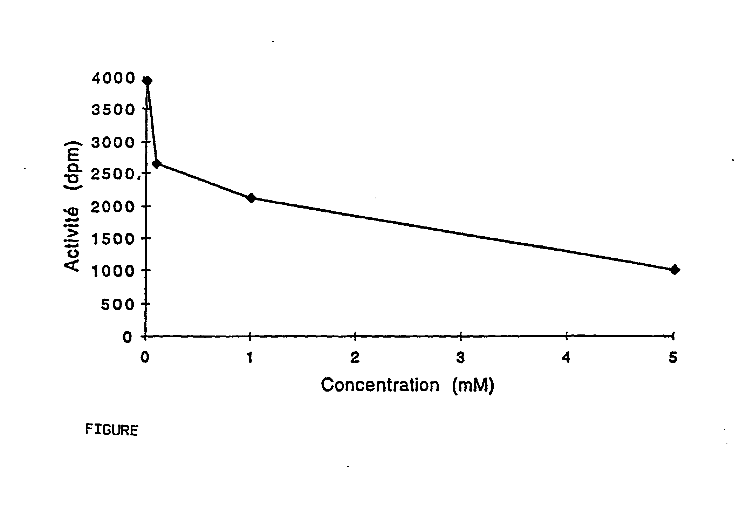

La figure illustre l'inhibition de l'activité de la lysyl-oxydase par un calix (6) arène selon l'invention ( n = 6; R₂ = H; R₁ = SO₃Na).

### EXEMPLE : Inhibition de la lysyl oxydase par un calix(6) arène selon l'invention (n = 6, R₂ = H; R₁ = SO₃Na).

### Matériels et méthodes.

### 1. Purification partielle de la lysyl-oxydase d'aorte bovine

Ce protocole est inspiré de celui de **(WILLIAMS et KAGAN,** Anal. Biochem. 149, 430-437, 1985), et de **(KAGAN et al.** Biochem. J. 177, 203-214 (1979)), qui a été sensiblement modifié.

Toutes les étapes sont effectuées à 4 °C ou dans la glace.

De l'aorte bovine foetale broyée est rincée par du tampon NaCI 0,12M, NaPi 16 mM, pH 7,7 puis par du NaPi 16 mM, pH 7,7 (tampon PB). La lysyl oxydase est extraite du résidu par de l'urée 4 M (concentration finale dans le tampon PB). Le surnageant obtenu après centrifugation à 10.000 g pendant 30 minutes est dilué deux fois dans du PB puis incubé avec de la DEAE-cellulose pendant 3 heures. La résine est mise en colonne et rincée successivement par du tampon PB contenant de l'urée 2M , du tampon PB puis du tampon PB contenant du NaCI 0,4 M.

La lysyl oxydase est éluée à partir de la DEAE-cellulose par de l'urée 6M dans du tampon PB. Les fractions présentant une absorbance à 280 nm sont réunies et dialysées pendant une nuit contre du tampon PB en présence de gel de Bleu de Cibacron (Biorad). Le gel est ensuite mis en colonne, et après des rinçages successifs par du tampon PB, du tampon PB-NaCI 1M et à nouveau du tampon PB, la lysyl oxydase est éluée du gel de Bleu de Cibacron par de l'urée 6 M dans du tampon PB.

L'éluat, appelé EB1 est centrifugé pour éliminer les agrégats, filtré (0,45 µm) puis concentré par ultrafiltration avec une membrane ayant un seuil de 10.000 Da. Cette fraction concentrée (1,7 mg/ml de protéines) est diluée dans différents milieux pour le dosage de l'activité.

### 2) Dosage de l'activité de la lysyl-oxydase

Le dosage a été effectué comme décrit par **CRONSHAW et al.** (Matrix 13, 255-266, 1993) et **SHACKLETON et HULMES** (Anal Biochem., 185, 359-362,1990), d'une manière sensiblement modifiée.

100 µl de ³H élastine suspendus dans un tampon de dosage (0,1M Na₂B₄O₇, 0,15 M NaCI, pH 8,0) sont mis dans un tube de microcentrifugeuse. 700 µl du tampon de dosage, et 100 µl de lysyl oxydase sont ajoutés. Le milieu réactionnel est agité à l'aide d'un vortex, puis incubé à 37°C durant des durées variables. Après incubation, la réaction est arrêtée en plaçant le tube de microcentrifugeuse dans de la glace puis en ajoutant 100 µl, d'acide trichloracétique à 50% (Poids/Volume). L'élastine est ensuite récupérée par centrifugation à 12.000 g durant 5 minutes à 2°C. 700 µl du surnageant sont transférés dans un microconcentrateur (Amicon Microcon 3), qui est centrifugé à 14.000 g durant 2 heures à 20°C . Durant la centrifugation les molécules de faible poids moléculaire passent à travers la membrane d'ultrafiltration (seuil de 3.000 kDa) et sont récupérés dans la chambre inférieure du microconcentrateur.

Afin de mesurer le relarguage du tritium, 400 µl de l'ultrafiltrat sont mélangés avec 3,6 ml du réactif « Aquasafe 300 Plus » commercialisé par la Société Zineser Analytic ( MAIDENHEAD, Grande-Bretagne), puis le comptage est effectué dans un compteur à scintillation liquide durant 10 minutes.

### Résultats:

La figure illustre l'inhibition de l'activité lysyl-oxydase par le calix(6)arène (n = 6, R¹ = SO₃Na, R₂ = H). Ce produit est obtenu par mise en solution dans un tampon adéquat du sulfonate (n = 6, R₁= SO₃H, R₂= H) commercialisé par la Société ACROS (GEEL WEST , Belgique) sous la référence 29.026-0010.

La concentration en calix(6)arène, nécessaire pour inhiber 50% de l'activité de lysyl-oxydase est de 1,2 mM.

## Revendications

1. Utilisation des calix(n)arènes pour la fabrication d'un médicament pour le traitement des maladies fibrotiques.

2. Utilisation selon la revendication 1 **caractérisée en ce que** les calix(n)arènes inhibent l'activité lysyl oxydase.

3. Utilisation selon l'une des revendications 1 et 2 **caractérisée en ce que** les calix(n)arènes présentent la formule I suivante: 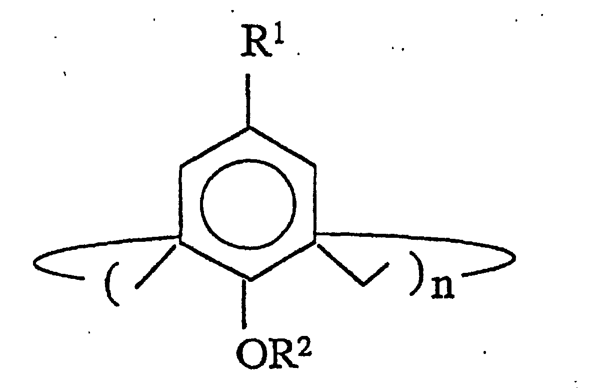 dans laquelle:
- R₁ représente un groupe alkyle, alkylamide, acyle, aryle, carboxyle, sulfonyle ou phosphonyle, éventuellement substitué,
- R₂ représente un atome d'hydrogène, un groupe alkyle, un groupe acyle ou un groupe amide, éventuellement substitué,
- n est un nombre entier compris entre 4 et 8, et leurs sels et esters pharmaceutiquement acceptables.

4. Utilisation selon la revendication 3, **caractérisée en ce que** R₁ représente un groupe SO₃H.

5. Utilisation selon l'une des revendications 3 et 4 **caractérisée en ce que** n est égal à 6.

6. Utilisation selon l'une des revendications 3 à 5 **caractérisée en ce que** R₂ est un atome d'hydrogène.

7. Utilisation selon l'une des revendications 3 à 6 **caractérisée en ce que** le calix-n-arène est tel que n est égal à 6, R₁ est un groupe SO₃H et R₂ est un atome d'hydrogène.

8. Utilisation selon l'une des revendications 3 à 7 **caractérisée en ce que** le sel pharmaceutiquement acceptable est un sel de lithium, de sodium, de potassium, de rubidium ou de césium.

9. Procédé in vitro d'inhibition de l'activité lysyl oxydase comprenant la mise en contact de la lysyl oxydase et d'un calix-n-arène:

10. Procédé selon la revendication 9 **caractérisé en ce que** le calix-n-arène présente la formule I selon la revendication **3.**

11. Utilisation in vitro des calix(n)arènes pour inhiber l'activité lysyl oxydase.

## Patentansprüche

1. Verwendung von Calix[n]arenen zur Herstellung eines Medikaments zur Behandlung fibrotischer Krankheiten.

2. Verwendung gemäß Anspruch 1, **dadurch gekennzeichnet, dass** die Calix[n]arene die Lysyl-Oxidase-Aktivität hemmen.

3. Verwendung gemäß einem der Ansprüche 1 und 2, **dadurch gekennzeichnet, dass** die Calix[n]arene die folgende Formel I aufweisen: 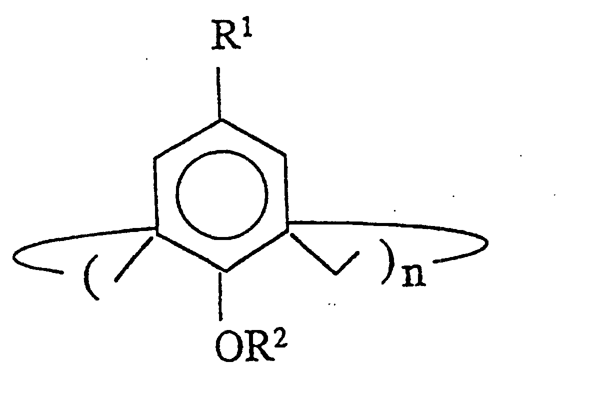 wobei
- R₁ eine gegebenenfalls substituierte Alkyl-, Alkylamid-, Acyl-, Aryl-, Carboxy-, Sulfonyl- oder Phosphonylgruppe darstellt;
- R₂ ein Wasserstoffatom, oder eine gegebenenfalls substituierte Alkylgruppe, Acylgruppe oder Amidgruppe darstellt;
- n eine ganze Zahl zwischen 4 und 8 ist;
und ihre pharmazeutisch annehmbaren Salze und Ester.

4. Verwendung gemäß Anspruch 3, **dadurch gekennzeichnet, dass** R₁ eine SO₃H-Gruppe darstellt.

5. Verwendung gemäß einem der Ansprüche 3 und 4, **dadurch gekennzeichnet, dass** n gleich 6 ist.

6. Verwendung gemäß einem der Ansprüche 3 bis 5, **dadurch gekennzeichnet, dass** R₂ ein Wasserstoffatom ist.

7. Verwendung gemäß einem der Ansprüche 3 bis 6, **dadurch gekennzeichnet, dass** das Calix[n]aren eines ist, bei dem n gleich 6 ist, R₁ eine SO₃H-Gruppe ist und R₂ ein Wasserstoffatom ist.

8. Verwendung gemäß einem der Ansprüche 3 bis 7, **dadurch gekennzeichnet, dass** das pharmazeutisch annehmbare Salz ein Lithium-, Natrium-, Kalium-, Rubidium- oder Cäsiumsalz ist.

9. In-vitro-Verfahren zur Hemmung der Lysyl-Oxidase-Aktivität, das das In-Kontakt-Bringen der Lysyl-Oxidase mit einem Calix[n]aren umfasst.

10. Verfahren gemäß Anspruch 9, **dadurch gekennzeichnet, dass** das Calix[n]-aren die Formel I gemäß Anspruch 3 aufweist.

11. Verwendung von Calix[n]arenen in vitro zur Hemmung der Lysyl-Oxidase-Aktivität.

## Claims

1. Use of calix(n)arenes in the manufacture of a medicament for treating fibrotic diseases.

2. Use according to claim 1 **characterized in that** the calix(n)arenes inhibit lysyl oxidase activity.

3. Use according to one of claims 1 and 2 **characterized in that** the calix(n)arenes have the formula I as follows: 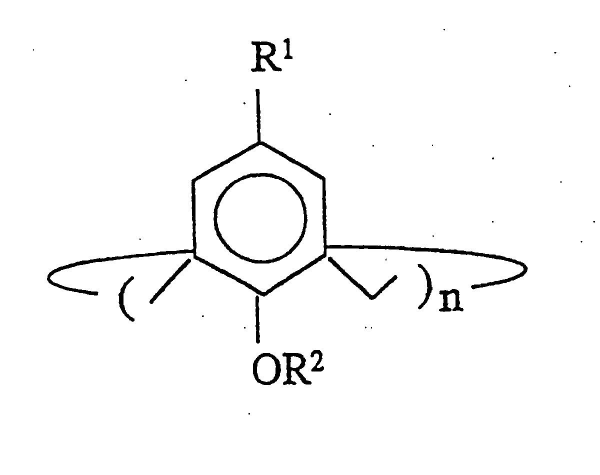 wherein
- R₁ represents an alkyl, alkylamide, acyl, aryl, carboxyl, sulfonyl or phosphonyl group, optionally substituted
- R₂ represents a hydrogen atom, or an alkyl, acyl or amide group, optionally substituted,
- n is an integer between 4 and 8, and their pharmaceutically acceptable salts and esters.

4. Use according to claim 3, **characterized in that** R₁ represents a SO₃H group.

5. Use according to one of claims 3 and 4, **characterized in that** n is equal to 6.

6. Use according to one of claims 3 to 5, **characterized in that** R₂ is a hydrogen atom.

7. Use according to one of claims 3 to 6, **characterized in that** the calix(n)arene is such that n is equal to 6, R₁ is a SO₃H group and R₂ is a hydrogen atom.

8. Use according to one of claims 3 to 7, **characterized in that** the pharmaceutically acceptable salt is a lithium, sodium, potassium, rubidium or cesium salt.

9. Method in vitro for inhibiting lysyl oxidase activity comprising bringing the lysyl oxidase into contact with a calix(n)arene.

10. Method according to claim 9 **characterized in that** the calix(n)arene has the formula I according to claim 3.

11. Use in vitro of calix(n)arenes to inhibit lysyl oxidase activity.
